# EUROPEAN PATENT APPLICATION

(11) **EP 3 355 052 A1**
(43) Date of publication of application: **01.08.2018**
(21) Application number: 17789061.3
(22) Date of filing: 28.02.2017
(51) Int. Cl.: G01N 27/22

(54) **SENSOR**

(30) Priority: 27.04.2016 JP 2016089099
(71) Applicant: KYB Corporation, Tokyo 105-6111 (JP)
(72) Inventor: YOSHIDA, Takahiro, Tokyo 105-6111 (JP); KAMEDA, Yukinori, Tokyo 105-6111 (JP)
(74) Representative: Horn Kleimann Waitzhofer Patentanwälte PartG mbB
(86) International application number: PCT/JP2017/007848
(87) International publication number: WO 2017/187772

(57) **Abstract**

A sensor is provided configured to appropriately detect the state of the fluid while reducing the effects of a fluid temperature on fluid characteristics. A sensor (1) includes a characteristics acquisition unit (10), a temperature measurement unit (20), a storage unit (30), and a correction unit (40). The characteristics acquisition unit (10) acquires a characteristic value for an in-device fluid. The temperature measurement unit (20) measures the in-device fluid temperature when the characteristics acquisition unit (10) acquires the characteristic value. The storage unit (30) stores a database registering an association between a pre-acquired characteristic of the in-device fluid and the in-device fluid temperature measured when the characteristic of the in-device fluid was pre-acquired. The correction unit (40) corrects the characteristic value of the in-device fluid acquired by the characteristics acquisition unit (10) based on the in-device fluid temperature measured by the temperature measurement unit (20) and the database.

## Description

### Technical Field

The present invention relates to a sensor.

### Background Art

A technique for detecting a state of a fluid has been proposed (see Patent document 1, for example).

An oil degradation detection apparatus is described in Patent document 1, configured to judge whether or not degradation has occurred in oil that is supplied to a rotating member or a sliding member in a circulating manner so as to allow such a member to operate smoothly while suppressing abrasion. The oil degradation detection apparatus includes a pair of electrode plates arranged in parallel with each other along an oil flow path. By applying an AC voltage across the pair of electrode plates, the oil degradation detection apparatus acquires the conductivity and the dielectric constant of the oil, and judges whether or not oil degradation has occurred based the conductivity and the dielectric constant thus acquired.

### Citation List

### Patent Literature

[Patent Document 1] Japanese Patent Application Laid Open No. 2009-2693

### Summary of Invention

### Technical Problem

In some cases, the fluid characteristics such as the electroconductivity, dielectric constant, etc., change according to the temperature of the fluid. Accordingly, in a case in which the fluid state is detected based on the fluid characteristics giving no consideration to the temperature of the fluid, this has the potential to involve a problem of not being able to appropriately detect the fluid state.

The present invention has been made in order to solve the aforementioned problem. Accordingly, it is a purpose of the present invention to provide a sensor that appropriately detects the fluid state while reducing the effects of the fluid temperature on the fluid characteristics.

### Solution to Problem

At least one embodiment of the present invention relates to a sensor that detects a state of a fluid in a device. The sensor comprises: a characteristics acquisition unit that acquires a value of characteristics of the fluid in the device; a temperature measurement unit that measures a temperature of the fluid in the device when the value of characteristics was acquired by the characteristics acquisition unit; a storage unit that stores a database as a registered information with respect to an association between the value of characteristics of the fluid in the device acquired beforehand and the temperature of the fluid in the device measured when the value of characteristics of the fluid in the device was acquired beforehand; and a correction unit that corrects the value of characteristics of the fluid in the device acquired by the characteristics acquisition unit based on the temperature of the fluid in the device measured by the temperature measurement unit and the database.

### Brief Description of the Drawings

Fig. 1 is a block diagram showing a sensor according to an embodiment of the present invention.
Fig. 2 is a schematic configuration diagram showing a characteristics acquisition unit included in the sensor according to an embodiment of the present invention.
Fig. 3 is a diagram for describing a storage unit included in the sensor according to an embodiment of the present invention.
Fig. 4 is a diagram for describing the storage unit included in the sensor according to an embodiment of the present invention.
Fig. 5 is a diagram for describing a correction unit included in the sensor according to an embodiment of the present invention.
Fig. 6 is a diagram for describing the correction unit included in the sensor according to an embodiment of the present invention.
Fig. 7 is a diagram for describing the correction unit included in the sensor according to an embodiment of the present invention.
Fig. 8 is a flowchart showing an operation of the sensor according to an embodiment of the present invention.
Fig. 9 is a flowchart showing an operation of the sensor according to an embodiment of the present invention.

### Description of Embodiments

Description will be made below regarding an embodiment of the present invention with reference to the drawings. It should be noted that a component or element of the following embodiment may be substituted by a different existing component or element as appropriate. Also, various kinds of variations may be made including a combination with a different existing component or element. Thus, description of the following embodiment is by no means intended to restrict the scope of the invention described in the appended claims.

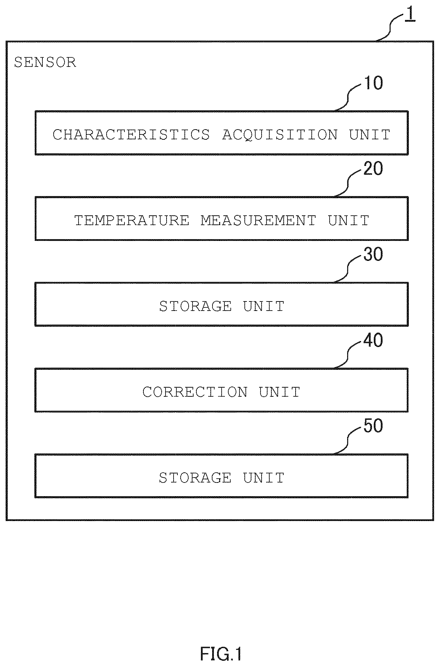

Fig. 1 is a block diagram showing a sensor 1 according to an embodiment of the present invention. The sensor 1 includes a characteristics acquisition unit 10, a temperature measurement unit 20, a storage unit 30, a correction unit 40, and a detection unit 50, and is configured to detect the state of lubricant oil in a device. The characteristics acquisition unit 10 acquires the dielectric constant of the lubricant oil in the device. The temperature measurement unit 20 measures the temperature of the lubricant oil in the device when the characteristics acquisition unit 10 acquires the dielectric constant. The storage unit 30 stores a database including registered information with respect to the association between the dielectric constant of the lubricant oil in the device acquired beforehand and the temperature acquired when the dielectric constant of the lubricant oil in the device was acquired. The correction unit 40 corrects the dielectric constant of the lubricant oil in the device acquired by the characteristics acquisition unit 10 based on the temperature of the lubricant oil in the device measured by the temperature measurement unit 20 and the database stored in the storage unit 30. The detection unit 50 detects the state of the lubricant oil based on the dielectric constant corrected by the correction unit 40.

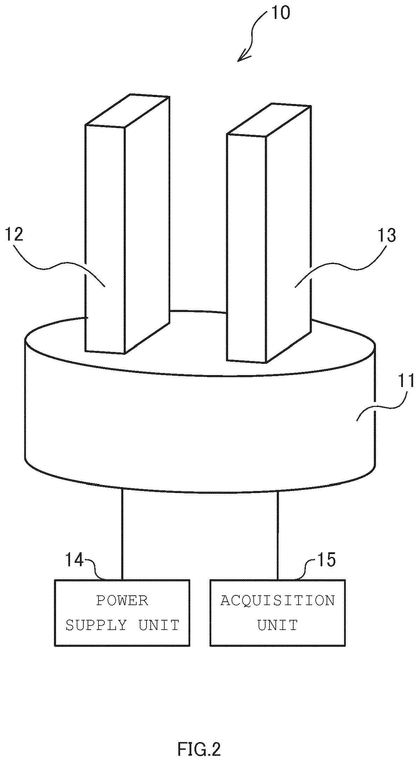

Fig. 2 is a schematic configuration diagram showing the characteristics acquisition unit 10. The characteristics acquisition unit 10 includes a circular base 11, a pair of electrodes 12 and 13 arranged such that they stand erect on the base 11, a power supply unit 14, and an acquisition unit 15.

The electrodes 12 and 13 are each configured as a planar-shaped electrode plate, and are arranged in parallel with each other. The power supply unit 14 applies an AC voltage across the electrodes 12 and 13 arranged in the lubricant oil. The acquisition unit 15 acquires the dielectric constant of the lubricant oil interposed between the electrodes 12 and 13 in a state in which the AC voltage is applied across the electrodes 12 and 13 by means of the power supply unit 14.

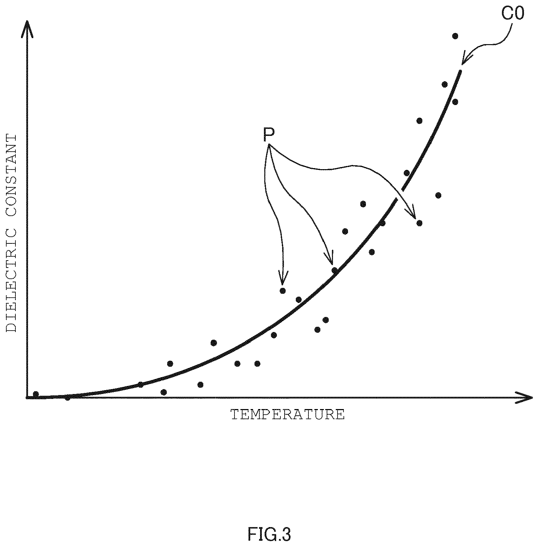

Description will be made below with reference to Fig. 3 regarding the database stored in the storage unit 30. In Fig. 3, the vertical axis represents the dielectric constant, and the horizontal axis represents the temperature.

In Fig. 3, the information with respect to the association between the dielectric constant of the lubricant oil in the device and the temperature is plotted as measurement data P. Furthermore, an approximated curve CO of the multiple measurement data P is shown.

With the information (measurement data P) with respect to the association between the dielectric constant of the lubricant oil in the device acquired beforehand and the temperature of the lubricant oil in the device measured when the dielectric constant of the lubricant oil in the device was acquired, two kinds of the aforementioned information are registered in the database.

One is the information with respect to the association between the dielectric constant of the lubricant oil in the device and the temperature registered beforehand at the point in time when the sensor 1 was manufactured.

The other is the information with respect to the association between the dielectric constant of the lubricant oil in the device acquired by means of the characteristics acquisition unit 10 in a predetermined period of time (which will simply be referred to as the "initial period" hereafter) that elapses from the time point at which the lubricant oil was charged to the device and the temperature of the lubricant oil in the device measured by the temperature measurement unit 20 when the dielectric constant was acquired by the characteristics acquisition unit 10.

That is to say, the information with respect to the association between the dielectric constant of the lubricant oil in the device and the temperature is registered in the aforementioned database at the point in time when the sensor 1 is manufactured. Subsequently, the information with respect to the association between the dielectric constant of the lubricant oil in the device and the temperature thereof acquired in the initial period is newly registered in the database.

Furthermore, the aforementioned database stores the information with respect to an approximated curve that represents the relation between the dielectric constant of the lubricant in the device and the temperature thereof calculated by the correction unit 40 based on the information (measurement data P) with respect to the association between the dielectric constant of the lubricant oil in the device and the temperature thereof registered in the database.

It should be noted that the aforementioned approximated curve may be designed in the form of a linear function, an n-th (n represents a desired integer of 2 or more) order function, an exponential function, a power function, or the like.

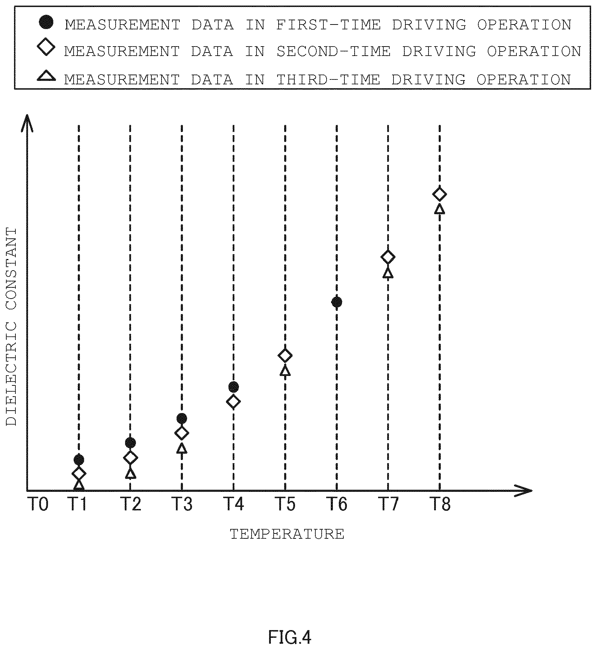

Description will be made below with reference to Fig. 4 regarding an example in which the measurement data and the approximated curve are registered in the database. In Fig. 4, the vertical axis represents the dielectric constant, and the horizontal axis represents the temperature.

It should be noted that Fig. 4 shows an example in which the information with respect to the association between the dielectric constant of the lubricant oil in the device and the temperature thereof is not registered at the point in time when the sensor 1 was manufactured.

Fig. 4 shows three kinds of measurement data acquired in the initial period. The solid circles represent the measurement data in the initial period when the device has been driven for the first time after the lubricant oil was charged to the device, i.e., the measurement data acquired in the initial period in the first-time driving operation after the lubricant oil was charged to the device. The rhombic symbols represent the measurement data acquired in the initial period in the second-time driving operation after the lubricant oil was charged to the device. The triangular symbols represent the measurement data acquire in the initial period in the third-time driving operation after the lubricant oil was charged to the device.

In the first-time driving operation, the temperature of the lubricant oil rises only up to a maximum of the temperature T6, and accordingly, the dielectric constant of the lubricant oil is not acquired at the temperature points T7 and T8. Furthermore, the temperature of the lubricant oil suddenly rises in a range from the temperature T4 to the temperature T6, and accordingly, the dielectric constant of the lubricant oil is not acquired at the temperature T5.

Thus, in the stage when the first-time driving operation has ended, the measurement values of the dielectric constant of the lubricant oil acquired at the temperature points T1 through T4 and T6 are registered as the measurement data in the database. Furthermore, an approximate curve is calculated in a range from the temperature T0 up to T6 based on the measurement data acquired in the first-time driving operation. The information with respect to the approximated curve thus calculated is also registered in the database.

It should be noted that, in the calculation of the approximated curve in a range from the temperature T0 up to T6, the correction unit 40 may calculate the dielectric constant of the lubricant oil for the temperature T5 by means of interpolation as described later. In this case, the approximated curve is calculated based on the measurement data of the dielectric constant of the lubricant oil acquired at the temperature points T1 through T4 and T6 in the first-time driving operation and the calculated value of the dielectric constant of the lubricant oil calculated for the temperature T5 by means of interpolation.

In the second-time driving operation, the measurement values of the dielectric constant of the lubricant oil are acquired at the temperature points T1 through T5, and T7 and T8.

Thus, in the stage when the second-time driving operation has ended, the measurement values of the dielectric constant of the lubricant oil acquired at the temperature points T1 through T5 and T7 and T8 are registered as additional measurement data in the database. Furthermore, an approximated curve is acquired in a range from the temperature points T0 to T8 based on the measurement data acquired in the first-time driving operation and the second-time driving operation. The information with respect to the approximated curve registered in the database is updated to the information with respect to the approximated curve acquired in the stage when the second-time driving operation has ended.

In the third-time driving operation, the measurement values of the dielectric constant of the lubricant oil are acquired at the temperature points T1 through T3 and T5, T7, and T8.

Thus, in the stage when the third-time driving operation has ended, the measurement values of the dielectric constant of the lubricant oil acquired at the temperature points T1 through T3 and T5, T7, and T8 are registered as additional measurement data in the database. Furthermore, an approximated curve is acquired in a range from the temperature points T0 to T8 based on the measurement data acquired in the first-time driving operation, the second-time driving operation, and the third-time driving operation. The information with respect to the approximated curve registered in the database is updated to the information with respect to the approximated curve acquired in the stage when the third-time driving operation has ended.

By calculating the approximated curve as described above, the correction unit 40 is capable of acquiring the relation between the dielectric constant of the lubricant oil and the temperature thereof in the initial period, i.e., the relation between the dielectric constant of the lubricant oil and the temperature thereof in a stage before the state of the lubricant oil changes due to foreign material contamination, degradation of the lubricant oil itself, or the like. Furthermore, the correction unit 40 corrects the dielectric constant of the lubricant oil acquired by the characteristics acquisition unit 10, using the approximated curve, to a corrected value of the dielectric constant of the lubricant oil for the predetermined reference temperature.

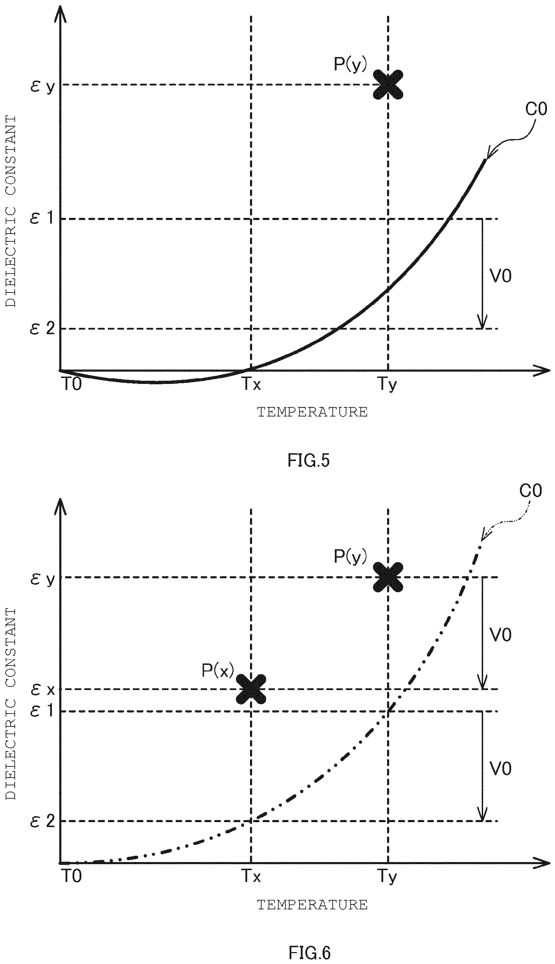

Description will be made below with reference to Figs. 5 and 6 regarding the correction of the dielectric constant of the lubricant oil by means of the correction unit 40. In Figs. 5 and 6, the vertical axis represents the dielectric constant, and the horizontal axis represents the temperature. The point P(y) represents the measurement data to be corrected by the correction unit 40, i.e., the point plotted as the information with respect to the association between the dielectric constant εy of the lubricant oil in the device acquired by the characteristics acquisition unit 10 and the temperature Ty of the lubricant oil in the device measured by the temperature measurement unit 20 when the dielectric constant εy was acquired by the characteristics acquisition unit 10. The temperature Tx represents the aforementioned reference temperature.

The point P(y) is not positioned on the approximated curve C0. This is because, in a case in which the state of the lubricant oil has changed due to foreign material contamination, degradation of the lubricant oil itself, or the like, this leads to a change in the dielectric constant of the lubricant oil. This means that the state of the lubricant oil at the time point at which the dielectric constant εy and the temperature Ty were acquired has changed from the state thereof at the time point at which the lubricant oil was charged to the device.

However, in a case of measuring the state of the same lubricant oil, the change in the dielectric constant of the lubricant oil per unit of temperature remains unchanged, i.e., the slope of the approximated curve that represents the relation between the dielectric constant of the lubricant oil and the temperature thereof remains unchanged even if the state of the lubricant oil has changed.

Thus, in a case in which the dielectric constant εy at the temperature Ty is to be corrected to the dielectric constant for the reference temperature Tx, first, as shown in Fig. 5, the correction unit 40 acquires the dielectric constant value ε1 at the temperature Ty and the dielectric constant value ε2 at the reference temperature Tx from the approximated curve C0. Next, the correction unit 40 calculates, based on the two values of the dielectric constant ε1 and ε2 thus acquired, the change in the dielectric constant of the lubricant oil due to the change in temperature from the temperature Ty to the reference temperature Tx in a stage in which a change in state due to foreign material contamination, degradation of the lubricant oil itself, or the like has not occurred, i.e., a theoretical value V0 of the change in the dielectric constant of the lubricant oil that occurs due to the change in temperature from the temperature Ty to the reference temperature Tx.

Next, as shown in Fig. 6, the correction unit 40 adds the theoretical value V0 to the dielectric constant εy represented by the point P(y), i.e., the dielectric constant εy of the lubricant oil in the device acquired by the characteristics acquisition unit 10, so as to calculate the dielectric constant value εx. The dielectric constant value εx is a corrected value obtained as a result of correcting the dielectric constant value εy of the lubricant oil at the temperature Ty to the dielectric constant value of the lubricant oil for the reference temperature Tx.

It should be noted that the correction unit 40 calculates the dielectric constant of the lubricant oil in a temperature range from the temperature measured by the temperature measurement unit 20 up to the reference temperature Tx by means of interpolation or otherwise extrapolation based on the measurement data P registered in the database.

The aforementioned interpolation means calculation of missing values between the values of the known numerical data string, based on the known numerical data string, or otherwise means the provision of a function that represents such missing values. Such a method is referred to as the "interpolation method (interpolating method)". On the other hand, the aforementioned extrapolation means calculation of anticipated values outside the range of the known numerical data, based on the known numerical data. Such a method is referred to as the "extrapolation method (extrapolating method)".

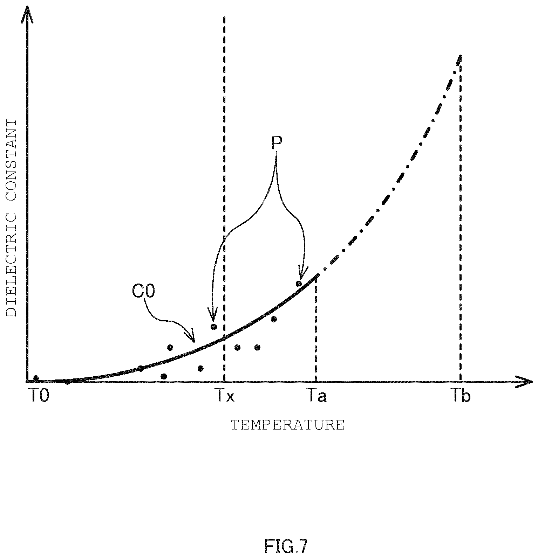

For example, the extrapolation provided by the correction unit 40 will be described below with reference to Fig. 7.

In Fig. 7, the vertical axis represents the dielectric constant, and the horizontal axis represents the temperature. In Fig. 7, the values of the dielectric constant in a temperature range of the temperature T0 to the temperature Ta are registered in the database. Furthermore, the information with respect to the approximated curve C0 that represents the relation between the dielectric constant of the lubricant oil and the temperature thereof is registered in the database. However, in a temperature range from the temperature Ta up to the temperature Tb, neither the values of the dielectric constant nor the approximated curve is registered in the database.

After the dielectric constant of the lubricant oil has been acquired at the temperature Tb by means of the characteristics acquisition unit 10, in a case in which the dielectric constant value thus acquired is to be corrected to a corrected value of the dielectric constant of the lubricant oil for the reference temperature Tx, first, the correction unit 40 reads out, from the database, the measurement data P registered in the database that represents the association between the dielectric constant of the lubricant oil and the temperature thereof. Next, at least one value is calculated for the dielectric constant of the lubricant oil in a temperature range from the temperature Tb to the temperature Ta by means of extrapolation based on the measurement data P thus read out. Next, an approximated curve is calculated in a range from the temperature Tb up to the temperature T0 based on the measurement data P read out from the database and the relation between the dielectric constant and the temperature calculated by means of extrapolation. Next, the information with respect to the approximated curve C0 registered in the database is updated based on the approximated curve thus calculated. Next, the dielectric constant of the lubricant oil acquired by the characteristics acquisition unit 10 is corrected using the approximated curve thus calculated, to a corrected value of the dielectric constant of the lubricant oil for the predetermined reference temperature.

The temperature measurement unit 20 described above is provided using an element such as a thermistor or an apparatus configured to measure the temperature. On the other hand, the characteristics acquisition unit 10, the storage unit 30, the correction unit 40, and the detection unit 50 are provided using a CPU, memory (RAM), hard disk, etc.

The hard disk stores an operating system, a program for executing a sequence of operations for acquiring and correcting the dielectric constant of the lubricant oil, etc. It should be noted that the hard disk may be configured as a non-temporary recording medium. For example, instead of such a hard disk, nonvolatile memory such as EPROM or flash memory, CD-ROM, or the like may be employed.

The CPU reads out the data and programs stored in the hard disk as appropriate, using the memory as appropriate, and executes calculation and processing as appropriate.

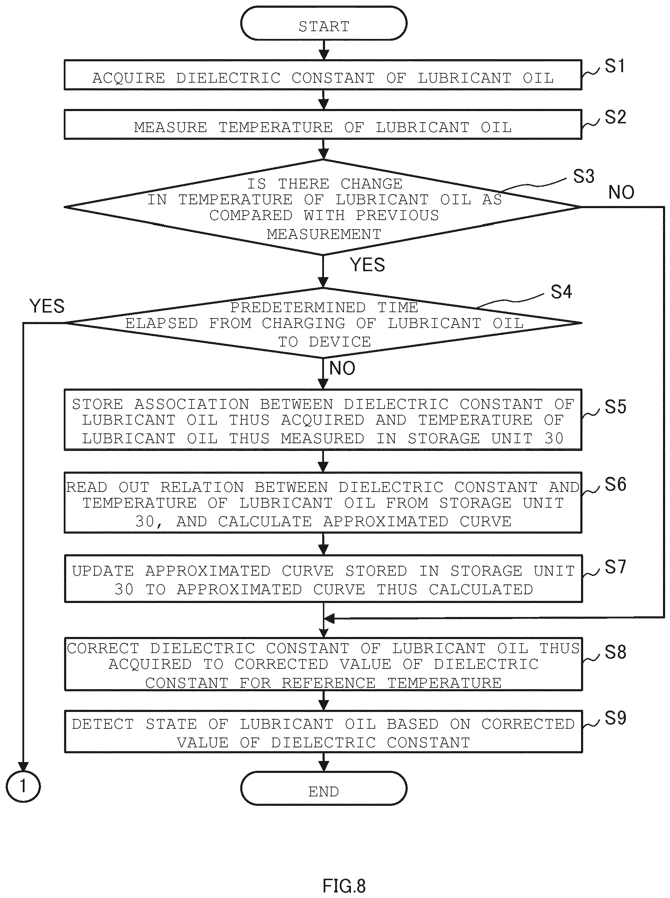

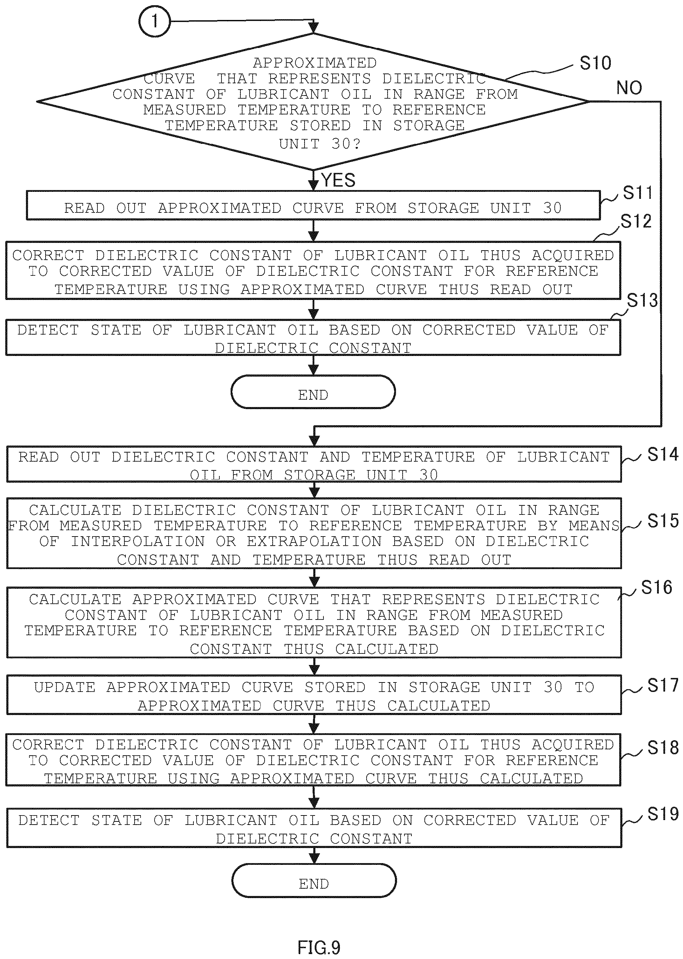

Figs. 8 and 9 are flowcharts showing operations of the sensor 1. The sensor 1 executes the operations from Step S1 shown in the flowcharts in Figs. 8 and 9.

In Step S1, the sensor 1 instructs the characteristics acquisition unit 10 to acquire the dielectric constant of the lubricant oil in the device. Subsequently, the operation transits to Step S2.

In Step S2, the sensor 1 instructs the temperature measurement unit 20 to measure the temperature of the lubricant oil in the device. Subsequently, the operation transits to Step S3.

In Step S3, the sensor 1 instructs the correction unit 40 to judge whether or not a change has occurred in the temperature of the lubricant oil as compared with the previous measurement timing. Specifically, in Step S2, when the difference in the temperature between the current measurement of the temperature of the lubricant oil and the previous measurement of the temperature of the lubricant oil is equal to or greater than a threshold value, judgment is made that a change has occurred. Conversely, when the difference in temperature between them is smaller than the threshold value, judgment is made that no change has occurred. With the sensor 1, in a case in which judgment is made by the correction unit 40 that there is a change, the operation transits to Step S4, and in a case in which judgment is made that there is no change, the operation transits to Step S8.

In Step S4, the sensor 1 instructs the correction unit 40 to judge whether or not a predetermined period of time has elapsed since the lubricant oil was charged to the device, i.e., whether or not the initial period has ended. When the sensor 1 has judged by means of the correction unit 40 that the initial period has ended, the sensor 1 switches the operation to Step S10 shown in Fig. 9. Conversely, when the sensor 1 has judged that the sensor 1 is operating in the initial period, the operation transits to Step S5.

It should be noted that, in order to judge whether or not such a predetermined period of time has elapsed since the lubricant oil was charged to the device, the sensor 1 measures the time elapsed since the lubricant oil was charged to the device. For example, when the lubricant oil in the device is replaced, the sensor 1 instructs the correction unit 40 to reset the elapsed time thus measured, according to a user's operation. Subsequently, the operation transits to Step S1.

In Step S5, the sensor 1 instructs the correction unit 40 to associate the values of the dielectric constant of the lubricant oil acquired in Step S1 with the measurement values of the temperature of the lubricant oil measured in Step S2, and register the association thus acquired in the database stored in the storage unit 30. Subsequently, the operation transits to Step S6.

In Step S6, the sensor 1 instructs the correction unit 40 to read out the measurement data P with respect to the association between the dielectric constant of the lubricant oil in the device and the temperature thereof from the database stored in the storage unit 30, and to calculate an approximated curve. Subsequently, the operation transits to Step S7.

In Step S7, the sensor 1 instructs the correction unit 40 to update the information with respect to the approximated curve registered in the database based on the approximated curve calculated in Step S6. Subsequently, the operation transits to Step S8.

In Step S8, the sensor 1 instructs the correction unit 40 to correct the dielectric constant of the lubricant oil acquired in Step S1 to a corrected value of the dielectric constant of the lubricant oil for the reference temperature Tx using the approximated curve calculated in Step S6. Subsequently, the operation transits to Step S9.

It should be noted that, when judgement has been made in Step S3 that there is no change, the operation transits to Step S8 without performing the operation in Step S6. In this case, in Step S8, the sensor 1 instructs the correction unit 40 to correct the dielectric constant of the lubricant oil acquired in Step S1 to a corrected value of the dielectric constant of the lubricant oil for the reference temperature Tx using the approximated curve calculated when the operation in Step S6 was performed in the previous sequence. Subsequently, the operation transits to Step S9.

In Step S9, the sensor 1 instructs the detection unit 50 to detect the state of the lubricant oil based on the corrected value of the dielectric constant calculated in Step S8. Subsequently, the operations shown in Figs. 8 and 9 end.

In Step S10 shown in Fig. 9, the sensor 1 instructs the correction unit 40 to judge whether or not the information with respect to the approximated curve that represents the dielectric constant of the lubricant oil in a range from the temperature measured in Step S2 up to the reference temperature Tx has been registered in the database stored in the storage unit 30. When the sensor 1 has judged by means of the correction unit 40 that the information has been registered, the sensor 1 switches the operation to Step S11. Otherwise, the sensor 1 switches the operation to Step S14.

In Step S11, the sensor 1 instructs the correction unit 40 to read out the approximated curve from the database stored in the storage unit 30. Subsequently, the operation transits to Step S12.

In Step S12, the sensor 1 instructs the correction unit 40 to correct the dielectric constant of the lubricant oil acquired in Step S1 to a corrected value of the dielectric constant of the lubricant oil for the reference temperature Tx using the approximated curve read out in Step S11. Subsequently, the operation transits to Step S13.

In Step S13, the sensor 1 instructs the detection unit 50 to detect the state of the lubricant oil based on the corrected value of the dielectric constant calculated in Step S12. Subsequently, the operations shown in Figs. 8 and 9 end.

In Step S14, the sensor 1 instructs the correction unit 40 to read out the measurement data P with respect to the association between the dielectric constant of the lubricant oil in the device and the temperature thereof from the database stored in the storage unit 30. Subsequently, the operation transits to Step S15.

In Step S15, the sensor 1 instructs the correction unit 40 to calculate the values of the dielectric constant of the lubricant oil in a range from the temperature measured in Step S2 up to the reference temperature Tx by means of interpolation or otherwise extrapolation based on the measurement data P read out in Step S14. Subsequently, the operation transits to Step S16.

In step S16, the sensor 1 instructs the correction unit 40 to calculate an approximated curve that represents the dielectric constant of the lubricant oil in a range from the temperature measured in Step S2 up to the reference temperature Tx based on the relation between the dielectric constant of the lubricant oil and the temperature thereof calculated in Step S15. Subsequently, the operation transits to Step S17.

In Step S17, the sensor 1 instructs the correction unit 40 to update the information with respect to the approximated curve registered in the database based on the approximated curve calculated in Step S16. Subsequently, the operation transits to Step S18.

In Step S18, the sensor 1 instructs the correction unit 40 to correct the dielectric constant of the lubricant oil acquired in Step S1 to a corrected value of the dielectric constant of the lubricant oil for the reference temperature Tx using the approximated curve calculated in Step S16. Subsequently, the operation transits to Step S19.

In Step S19, the sensor 1 instructs the detection unit 50 to detect the state of the lubricant oil based on the corrected value of the dielectric constant calculated in Step S18. Subsequently, the operations shown in Figs. 8 and 9 end.

As described above, the sensor 1 instructs the characteristics acquisition unit 10 to acquire the dielectric constant of the lubricant oil in the device. Furthermore, the sensor 1 instructs the temperature measurement unit 20 to measure the temperature of the lubricant oil in the device when the dielectric constant was acquired by means of the characteristics acquisition unit 10. The storage unit 30 stores, in the form of a registered database, the association between the dielectric constant of the lubricant oil in the device measured beforehand and the temperature of the lubricant oil in the device measured when the dielectric constant of the lubricant oil in the device was measured beforehand. The correction unit 40 corrects the dielectric constant of the lubricant oil acquired by the characteristics acquisition unit 10 based on the temperature of the lubricant oil measured by the temperature measurement unit 20 and the database stored in the storage unit 30. This allows the dielectric constant of the lubricant oil to be acquired giving consideration to the temperature of the lubricant oil. Accordingly, this is capable of reducing the effects of the temperature of the lubricant oil on the dielectric constant of the lubricant oil acquired by the characteristics acquisition unit 10, thereby allowing the state of the lubricant oil to be appropriately detected.

Furthermore, the sensor 1 instructs the correction unit 40 to correct the dielectric constant of the lubricant oil acquired by the characteristics acquisition unit 10 to a corrected value of the dielectric constant of the lubricant oil for the reference temperature Tx based on the temperature of the lubricant oil measured by the temperature measurement unit 20 when the dielectric constant was acquired by the characteristics acquisition unit 10 and the database stored in the storage unit 30. This is capable of estimating the value of the dielectric constant of the lubricant oil at the reference temperature Tx assuming that the dielectric constant of the lubricant oil measured by the characteristics acquisition unit 10 changes due to a change in temperature.

Furthermore, the sensor 1 instructs the correction unit 40 to calculate the theoretical value of the change in the dielectric constant of the lubricant oil due to a change in temperature from the temperature measured by the temperature measurement unit 20 to the reference temperature Tx. The correction unit 40 corrects the dielectric constant of the lubricant oil acquired by the characteristics acquisition unit 10 to a corrected value of the dielectric constant of the lubricant oil for the reference temperature Tx. This is capable of calculating the change in the dielectric constant of the lubricant oil due to a change in the temperature from the temperature measured by the temperature measurement unit 20 to the reference temperature in a stage before a change has occurred in the state of the lubricant oil itself. Furthermore, this is capable of correcting the dielectric constant of the lubricant oil acquired by the characteristics acquisition unit 10 based on the change in the dielectric constant thus calculated. This is capable of appropriately estimating the effects of the difference between the temperature measured by the temperature measurement unit 20 and the reference temperature Tx on the dielectric constant of the lubricant oil. Thus, this is capable of appropriately correcting the dielectric constant of the lubricant oil acquired by the characteristics acquisition unit 10.

Furthermore, the sensor 1 instructs the correction unit 40 to calculate the values of the dielectric constant of the lubricant oil in the device in a range from the temperature measured by the temperature measurement unit 20 up to the reference temperature Tx by means of interpolation or otherwise extrapolation based on the measurement data P registered in the database. Thus, by acquiring missing information by means of interpolation or otherwise extrapolation, this allows the correction unit 40 to perform the correction even if the information required for the correction to be performed by the correction unit 40 has not been registered in the database after the initial period ends.

In a case in which the information with respect to the dielectric constant of the lubricant oil registered in the database was acquired in an environment at a high temperature, e.g., in summer, this has the potential to involve a problem in that the information with respect to the dielectric constant of the lubricant oil registered in the database does not cover the dielectric constant of the lubricant oil in a state at a low temperature. Specifically, in a case in which the environmental temperature has become low in winter, for example, this has the potential to involve a problem in that the information registered in the database does not have data required for the correction unit 40 to perform the correction.

In order to solve such a problem, as described above, the sensor 1 instructs the correction unit 40 to calculate the values of the dielectric constant of the lubricant oil in the device in a range from the temperature measured by the temperature measurement unit 20 up to the reference temperature Tx by means of interpolation or otherwise extrapolation based on the measurement data P registered in the database. In a case in which a change occurs in the environment, the information required for the correction unit 40 to perform the correction is calculated, and the information thus calculated is registered as additional information in the database. Thus, this allows the state of the lubricant oil to be appropriately detected even in such a case.

Furthermore, the sensor 1 instructs the correction unit 40 to associate the measurement data of the dielectric constant of the lubricant oil acquired by the characteristics acquisition unit 10 with the measurement data of the temperature of the lubricant oil measured by the temperature measurement unit 20 measured when the dielectric constant was acquired by the characteristics acquisition unit 10, and to register the association thus acquired in the database stored in the storage unit 30. Even in a case in which the relation between the dielectric constant of the lubricant oil charged to the device and the temperature thereof has not been registered in the database beforehand, the relation between them can be acquired by means of the characteristics acquisition unit 10 and the temperature measurement unit 20, and the relation thus acquired can be newly registered in the database. Accordingly, this is capable of reducing the effects of the temperature of the lubricant oil even if the relation between the dielectric constant of the lubricant oil and the temperature thereof has not been registered in the database beforehand.

The state of the lubricant oil changes with the passage of time after the lubricant oil is charged to the device due to foreign material contamination, degradation of the lubricant oil itself, or the like. Accordingly, the dielectric constant of the lubricant oil also changes.

In order to solve such a problem, the sensor 1 performs the operation in which the association between the dielectric constant of the lubricant oil and the temperature thereof is registered in the database for the measurement values of the dielectric constant of the lubricant oil acquired by the characteristics acquisition unit 10 in the initial period. This allows the relation between the dielectric constant of the lubricant oil and the temperature thereof to be registered in the database in a stage when a change has not occurred in the state of the lubricant oil due to foreign material contamination, degradation of the lubricant oil itself, or the like.

Furthermore, when the lubricant oil in the device is replaced, for example, the sensor 1 instructs the correction unit 40 to reset the elapsed time measured from the time point at which the lubricant oil was charged to the device, according to the user's operation. Subsequently, the operation is returned to Step S1. In a case in which there is a difference in the kind of the lubricant oil between before and after the replacement of the lubricant oil in the device, the relation between the dielectric constant and the temperature is newly acquired, and the relation thus acquired is registered in the database. Thus, this allows the state of the lubricant oil to be appropriately detected even in such a case.

In a case in which only insufficient cleaning has been performed for the internal components of the device when the replacement of the lubricant oil is conducted, this has the potential to have an effect on the dielectric constant of the lubricant oil even in a case in which there is no difference in the kind of the lubricant oil between before and after the replacement of the lubricant oil in the device.

In order to solve such a problem, in a case in which the lubricant oil in the device has been replaced, for example, the sensor 1 instructs the correction unit 40 to reset the elapsed time according to a user's operation. Subsequently, the operation is returned to Step S1. With such an arrangement, after the replacement of the lubricant oil, the relation between the dielectric constant and the temperature is newly acquired giving consideration to the effects of the cleaning, and the relation thus acquired is registered in the database. Thus, this allows the state of the lubricant oil to be appropriately detected.

It should be noted that description has been made in the present embodiment regarding an arrangement in which, in Step S5 shown in Fig. 8, the sensor 1 associates the dielectric constant of the lubricant oil acquired in the Step S1 with the temperature of the lubricant oil measured in Step S2, and registers the association thus acquired in the database stored in the storage unit 30. That is to say, with the sensor 1 according to the present embodiment, the information with respect to the association between the dielectric constant of the lubricant oil and the temperature thereof acquired in the initial period is registered in the database. However, the present invention is not restricted to such an arrangement. Also, an arrangement may be made in which the information with respect to the association between the dielectric constant of the lubricant oil and the temperature thereof acquired in the initial period is not newly registered in the database.

Description has been made in the present embodiment regarding an arrangement in which the information associating the dielectric constant of the lubricant oil in the device and the temperature thereof has been registered beforehand at the point in time when the sensor 1 was manufactured. However, the present invention is not restricted to such an arrangement. That is to say, an arrangement may be made in which the information associating the dielectric constant of the lubricant oil in the device and the temperature thereof is not registered in the database beforehand at the point in time when the sensor 1 was manufactured. In this case, the information with respect to the association between the dielectric constant of the lubricant oil in the device and the temperature thereof acquired in the initial period may be newly registered in the database.

Description has been made in the present embodiment regarding an arrangement in which the dielectric constant of the lubricant oil is employed as the characteristic of the lubricant oil. However, the present invention is not restricted to such an arrangement. Also, the conductivity of the lubricant oil or a combination of the dielectric constant and the conductivity of the lubricant oil may be employed. Also, other characteristics of the lubricant oil that differ from the dielectric constant or the conductivity may be employed. Specifically, the viscosity of the lubricant oil may be employed.

Description has been made in the present embodiment regarding an arrangement in which the sensor 1 detects the state of the lubricant oil. However, the present invention is not restricted to such an arrangement. Also, the sensor 1 is able to provide fluid state detection regardless of the kind of fluid. For example, the sensor 1 is capable of detecting the contamination level of water, the quality of a chemical or drinking water, etc.

Description has been made in the present embodiment regarding an arrangement in which the planar-shaped electrodes 12 and 13 are arranged in parallel. However, the present invention is not restricted to such an arrangement. For example, the electrodes 12 and 13 may be formed in the form of a cylindrical shape or a cylindroid shape. Also, the electrodes 12 and 13 may be respectively formed in two cylindrical shapes having different inner diameters. Also, one of the electrodes 12 and 13 may be arranged in the internal space of the other with the central axes thereof aligned, as with a coaxial cylindrical capacitor, for example.

Description has been made with reference to the present embodiment. With this embodiment, this arrangement is capable of reducing the effects of the temperature on the characteristics of a fluid, thereby allowing the state of the fluid to be appropriately detected.

It should be noted that although detailed description has been made regarding the embodiment of the present invention with reference to the drawings, specific configurations thereof are not restricted to this embodiment. Rather, various changes of design may be made, which are encompassed by the present invention without departing from the spirit or scope of the appended claims.

Also, the present invention may be applicable to a hydraulic device, for example.

### Reference Signs List

1 sensor
10 characteristics acquisition unit
11 base
12, 13 electrode
14 power supply unit
15 acquisition unit
20 temperature measurement unit
30 storage unit
40 correction unit
50 detection unit.

## Claims

1. A sensor that detects a state of a fluid in a device, the sensor comprising:
a characteristics acquisition unit that acquires a value of characteristics of the fluid in the device;
a temperature measurement unit that measures a temperature of the fluid in the device when the value of characteristics was acquired by the characteristics acquisition unit;
a storage unit that stores a database as a registered information with respect to an association between the value of characteristics of the fluid in the device acquired beforehand and the temperature of the fluid in the device measured when the value of characteristics of the fluid in the device was acquired beforehand; and
a correction unit that corrects the value of characteristics of the fluid in the device acquired by the characteristics acquisition unit based on the temperature of the fluid in the device measured by the temperature measurement unit and the database.

2. The sensor according to claim 1, wherein the correction unit corrects the value of characteristics of the fluid in the device acquired by the characteristics acquisition unit to a corrected value of the characteristics of the fluid in the device for a predetermined reference temperature, based on the database and the temperature of the fluid in the device measured by the temperature measurement unit when the value of characteristics was acquired by the characteristics acquisition unit.

3. The sensor according to claim 2, wherein the correction unit calculates, based on the database, a theoretical value of a change in characteristics of the fluid in the device in a range from the temperature measured by the temperature measurement unit to the reference temperature,
and wherein the correction unit corrects the value of the characteristics of the fluid in the device acquired by the characteristics acquisition unit based on the theoretical value thus calculated, so as to obtain a corrected value of the characteristics of the fluid in the device for the reference temperature.

4. The sensor according to claim 3, wherein the correction unit calculates a value of characteristics of the fluid in a range from the temperature measured by the temperature measurement unit to the reference temperature by means of interpolation or otherwise extrapolation based on the information registered in the database.

5. The sensor according to claim 4, wherein the correction unit acquires, based on the value of characteristics of the fluid calculated by means of interpolation or otherwise extrapolation, an approximated curve that represents the relation between the characteristics of the fluid in the device and the temperature in a range from the temperature measured by the temperature measurement unit to the reference temperature,
wherein the correction unit registers the approximated curve thus acquired in the database,
and wherein the correction unit corrects the value of characteristics of the fluid in the device acquired by the characteristics acquisition unit using the approximated curve thus acquired, so as to obtain a corrected value of the characteristics of the fluid in the device for the reference temperature.

6. The sensor according to claim 1, wherein the correction unit associates the characteristics of the fluid in the device acquired by the characteristics acquisition unit in a predetermined period that elapses after the fluid was charged to the device with the temperature of the fluid in the device measured by the temperature measurement unit when the value of characteristics was acquired by the characteristics acquisition unit,
and wherein the correction unit registers the association thus acquired in the database.
